# FASCICULE DE BREVET EUROPEEN

(11) **EP 2 178 496 B1**
(45) Date de publication et mention de la délivrance du brevet: **16.03.2016**
(21) Numéro de dépôt: 08826887.5
(22) Date de dépôt: 16.07.2008
(51) Int. Cl.: A61K 8/44, A61K 8/49, A61K 8/67, A61Q 19/08, A61N 5/06, A61N 5/067

(54) **UTILISATION DE LUMIERE VERTE POUR ACTIVER LA L-AMINO ACIDE OXYDASE**
VERWENDUNG VON GRÜNEM LICHT ZUR AKTIVIERUNG VON L-AMINOSÄURE-OXIDASE
USE OF GREEN LIGHT TO ACTIVATE L-AMINO ACID OXYDASE

(30) Priorité: 16.07.2007 FR 0756506; 25.10.2007 US 982455 P
(43) Date de publication de la demande: 28.04.2010
(73) Titulaire: L'Oréal, 75008 Paris (FR)
(72) Inventeur: LABOUREAU, Julien, 92130 Issy Les Moulineaux (FR); NGUYEN, Quang Lan, F-92160 Antony (FR)
(74) Mandataire: Hugodot, Yannick
(86) Numéro de dépôt international: PCT/FR2008/051333
(87) Numéro de publication internationale: WO 2009/019381

(56) Documents cités:
- EP-A- 1 637 123
- EP-A- 1 726 296
- WO-A-2004/087167
- FR-A- 2 729 076
- FR-A- 2 895 680
- US-A- 5 061 480

## Description

La présente invention relève du domaine du soin de la peau et des cheveux et, en particulier, de l'amélioration de l'équilibre physiologique de la peau et des cheveux et la prévention et/ou le traitement de leur vieillissement.

La présente invention se rapporte plus spécifiquement à l'utilisation d'une source de lumière verte pour activer la L-amino acide oxydase en présence d'au moins un substrat de cette enzyme afin de stimuler le métabolisme énergétique des cellules de la peau, stimuler leur renouvellement et ainsi améliorer l'apparence de la peau et des cheveux, en particulier, prévenir et/ou traiter les signes cutanés du vieillissement de la peau et des cheveux.

L'invention se rapporte également à une composition comprenant au moins un substrat de l'enzyme et au moins une substance particulière capable d'émettre de la lumière verte, à un kit comprenant une composition à base d'une substance émettant de la lumière verte et une composition comprenant au moins un substrat de ladite enzyme et à un procédé cosmétique mettant en oeuvre les compositions ou kits selon l'invention.

La peau est un organe externe du corps constamment soumis aux agressions de l'environnement extérieur telles que les rayonnements du soleil, les polluants atmosphériques...

Ces facteurs extérieurs mais aussi le déclin progressif des différentes fonctions de l'organisme au cours du vieillissement concourent à une accumulation de composants cellulaires endommagés (acide nucléiques, lipides, protéines) dont l'élimination et/ou la réparation devrait être assurée par la cellule.

La formation de composants cellulaires endommagés s'effectue principalement par des réactions impliquant des espèces réactives de l'oxygène telles que l'anion superoxyde, le peroxyde d'hydrogène ou le radical hydroxyle. D'autres réactions impliquant la fixation sur les macromolécules de glucides ou d'aldéhydes issus de la péroxydation lipidique y contribuent également (Friguet B. Le vieillissement moléculaire et cellulaire et ses futurs enjeux, Mécanismes biochimiques).

Conserver une apparence jeune et/ou une peau saine conduit à la recherche incessante de nouveaux composés et/ou de nouvelles compositions permettant de maintenir ou d'améliorer l'apparence de la peau.

C'est ce à quoi est parvenu la Demanderesse en associant l'application d'une composition comprenant au moins un substrat de la L-amino acide oxydase et l'exposition à une lumière verte qui a pour effet d'activer l'enzyme L-amino acide oxydase.

Il est connu d'utiliser des rayons lumineux pour le traitement de la peau. A ce titre, on peut citer :
- les lasers qui sont des rayonnements électromagnétiques de longueur d'onde 532 nm et qui sont utilisés notamment pour traiter les phénomènes de télangiectasie ou d'hyperpigmentation liés à l'âge (Robert *et al.,* 2003). Plusieurs travaux ont montré que les lasers émis à la longueur d'onde de 1064 nm améliorent la régénération cutanée. Les lasers appliqués de façon pulsée à 585-595 nm améliorent nettement les rides localisées au niveau de la région périorbitale.
- L'IPL (intense pulsed light) : les rayonnements électromagnétiques à large spectre compris entre 500 et 1200 nm (IPL) ont été largement décrits pour améliorer les phénomènes de vieillissement. L'utilisation de filtres spécifiques permet d'isoler des longueurs d'ondes et ne conserver que les rayonnements électromagnétiques adaptés aux types de traitements souhaités. Il a été montré que l'émission simultanée de rayonnements jaunes rouges et infrarouges permet de réguler plusieurs activités biologiques différentes et ainsi de réguler diverses altérations liées au photo vieillissement.
- Les rayonnements infrarouges : plusieurs travaux ont montré l'effet d'une irradiation infrarouge à large spectre sur la biologie de la peau. Il a été décrit en particulier que l'absorption des infrarouges par les molécules d'eau du tissu induit une augmentation localisée de chaleur au niveau du derme entraînant une augmentation de synthèse de molécules matricielles et en particulier de collagène. Plus récemment il a été montré qu'une irradiation aux infrarouges protège les fibroblastes du derme de l'effet délétère d'une irradiation aux UV. Dans ces travaux les auteurs montrent que cette protection passe par la régulation d'une protéine mitochondriale, le cytochrome C (Menezes *et al.,* 1998)
- De plus en plus d'équipes scientifiques cherchent à déterminer le mécanisme d'action biologique de la LED. Il a été montré en particulier que la LED en ciblant des photorécepteurs (chromophores) active la chaîne d'oxydo réduction localisée au niveau de la membrane mitochondriale. Cette stimulation conduirait alors au niveau des fibroblastes à la production de protéines fibrillaires les collagènes et l'élastine et à la diminution de l'expression des métaloprotéinases MMPs (Robert *et al.,* 2003). Dans une étude récente, il a été montré par Vinck *et al.,* 2005 que l'irradiation par une source de LED à 570 nm induit une augmentation de la prolifération des fibroblastes.

La Demanderesse a découvert, de manière surprenante et inattendue, que l'application sur la peau d'un substrat de la L-amino acide oxydase accompagnée d'une émission de lumière verte conduisait à une activité accrue de cette enzyme et présentait des effets avantageux sur la stimulation et la régulation de l'activité physiologique de la peau.

La L-amino acide oxydase (EC 1.4.3.2) appartient à la famille des amino acide oxydases. Les enzymes amino acides oxydases sont présentes dans les différents types cellulaires de la peau.
Elles catalysent la transformation des fonctions amines en fonctions cétoniques. La transformation d'un acide aminé par ces enzymes donne donc naissance au composé ceto acide correspondant.
La demanderesse a maintenant mis en évidence que la lumière verte avait la propriété d'augmenter l'activité de la L-amino acide oxydase.

Les acides aminés sont des combustibles qui, après transformation dans le cytoplasme de la cellule, entrent dans les mitochondries pour y être dégradés.

L'alanine, en particulier, peut donner directement naissance au pyruvate par désamination oxydative catalytique avec la L-amino acide oxydase.

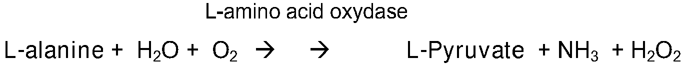

Dans la mitochondrie, le pyruvate est transformé en ATP, principale source énergétique de la cellule.

Parmi les composés ceto acides, on compte notamment le pyruvate, ou encore l'acétoacétate.

La formation de l'acétoacétate peut aussi être induite par désamination oxydative selon le même principe celui cité ci dessus à partir de l'amino butyrate.

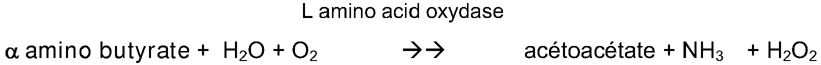

L'invention a donc pour objet l'utilisation de lumière verte pour activer la L-amino acide oxydase en présence d'au moins un de ses substrats.

Une telle utilisation a pour effet d'augmenter le potentiel de phosphorylation qui active des signaux conduisant à l'expression de gènes et d'activer le métabolisme des cellules de la peau, en particulier, des fibroblastes, des kératinocytes et des mélanocytes, d'activer et de réguler la physiologie de ces cellules notamment en diminuant la formation et en stimulant l'élimination de composants cellulaires endommagés, et particulier des composants résultant du stress oxydatif.

Une conséquence de cet effet est d'améliorer le renouvellement des cellules du derme et de l'épiderme et ainsi la régénération cutanée.

L'utilisation selon l'invention permet donc de donner à la peau et aux cheveux un aspect sain, c'est-à-dire unifier le teint, raviver son éclat, éviter son aspect jaune ou terne ; prévenir et/ou traiter les taches cutanées ; atténuer les irrégularités visibles et/ou tactiles de la surface de la peau, lui donner un relief plus uniforme et la lisser, prévenir et/ou traiter les rides et ridules ; redonner plus d'épaisseur et d'élasticité à la peau, en particulier, pour des zones où la peau est davantage relâchée, par exemple, sur le contour du visage ou dans le cou.

L'utilisation selon l'invention permet encore de lutter contre les altérations de la peau liées au vieillissement, de prévenir et/ou de traiter les signes cutanés du vieillissement de la peau et les signes du vieillissement des cheveux ; d'améliorer la cicatrisation.
En particulier, l'utilisation selon l'invention permet d'augmenter la synthèse des macromolécules matricielles du derme (fibrilles de collagène, GAG, fibres élastiques), et d'éviter leur dégradation, elle évite aussi l'altération de l'homéostasie épidermique.

Par signes cutanés du vieillissement de la peau, on entend notamment, un relâchement de la peau, l'apparition de rides, de taches cutanées, un jaunissement du teint.

Par signes du vieillissement des cheveux, on entend notamment la chute des cheveux, le ralentissement de leur pousse, leur grisonnement, la diminution de leur diamètre, de leur vigueur.

La mise en oeuvre de l'invention se fait par l'administration simultanée ou décalée d'une composition comprenant au moins un substrat de la L-amino acide oxydase et de la lumière verte sur la zone où ladite composition a été appliquée.

Les substrats de la L-amino acide oxydase utilisables selon l'invention sont les amino acides ou une molécule comprenant une fonction amine, le substrat étant tel que qu'il comporte un carbone en alpha du groupe acide et un groupe d'amine primaire, plus particulièrement, il s'agit des L-amino acides.

Ledit substrat est choisi parmi la leucine, la sérine, la thréonine, la glycine, l'alanine, la valine, la phénylalanine, la tyrosine, la lysine, l'arginine, la cystéine, l'aspartate, le glutamate, la citruline, l'ornithine, l'acide diaminopimelique, la penicilamine, l'acide α-amino butyrique.

De préférence, l'invention est mise en oeuvre avec l'alanine, la glycine, l'acide α-amino butyrique, la lysine, la leucine, la sérine et la thréonine.

Les acides aminés pourront être formulés sous forme de sels, tels que les sels sodiques ou alcalino-terreux (par exemple, manganèse ou calcium).

Selon un mode préféré, l'invention est mise en oeuvre à l'aide d'une composition topique comprenant au moins un substrat de la L-amino acide oxydase.

Par composition topique, on entend une composition destinée à une application locale sur toute surface du corps, incluant, la peau, les muqueuses ou semi-muqueuses, le cuir chevelu.
Les compositions utiles selon l'invention sont en particulier des compositions cosmétiques ou dermatologiques.

La composition comprend généralement un milieu physiologiquement acceptable, c'est-à-dire compatible avec la peau et/ou ses phanères. Il s'agit de préférence d'un milieu cosmétiquement acceptable, c'est-à-dire qui présente une odeur, une couleur et un toucher agréables et qui ne génère pas d'inconforts (picotements, rougeurs, tiraillements) inacceptables, susceptibles de détourner la consommatrice d'utiliser cette composition.

La composition topique selon l'invention comprend une quantité efficace de substrat (acide aminé) choisie aussi en fonction de la solubilité de l'acide aminé dans la formule considérée, cette quantité de substrat de la L-amino acide oxydase est préférentiellement comprise entre 0,001 à 10%, de façon encore, préférée entre 0,01 et 1%.

Selon la zone du corps visée et l'intensité d'application désirée, l'homme de métier pourra choisir parmi différentes formes de composition :
- une composition destinée à restée appliquée sur la peau même après l'exposition à la lumière verte, cette composition peut être une dispersion du type lotion ou gel, une émulsion de consistance liquide ou semi-liquide du type lait, obtenue par dispersion d'une phase grasse dans une phase aqueuse (H/E) ou inversement (E/H), ou une suspension ou émulsion de consistance molle, semi-solide ou solide du type crème ou gel, ou encore des émulsions multiples (E/H/E ou H/E/H), une microémulsion, une dispersion vésiculaire de type ionique et/ou non ionique, ou une dispersion cire/phase aqueuse ;
- une composition qui ne reste en contact avec la peau que pendant la durée de l'exposition à la lumière verte, telle qu'un masque, sous forme de crème que l'utilisateur peut appliquer spécifiquement sur les zones à traiter puis retirer ensuite, un patch imbibé d'un substrat ; sous cette forme d'administration, les masques ou patchs doivent être suffisamment transparents pour laisser passer la lumière verte.

Dans le cadre de la présente invention, toute source de lumière de couleur verte émettant à une longueur d'onde telle qu'elle active la L-amino-acide oxydase, pouvant être comprise entre 500 et 580 nm, de préférence entre 500 et 560 nm.

On peut notamment citer :
- les lasers (pour Light Amplification by the Stimulated Emission of Radiation), il s'agit d'une source lumineuse de très haute intensité et monochromatique.
A la différence de la lumière blanche (photons de diverses longueurs d'onde émis de façon anarchique à différents instants et dans des directions différentes), la radiation lumineuse émise par un laser est une lumière constituée de photons émis en même temps et dans la même direction.
Trois éléments caractérisent les lasers : la longueur d'onde (λ) ; le mode d'émission : continu (puissance délivrée constante), pulsé (l'énergie est délivrée par pulses dont la fréquence et la puissance sont modulables), et ultra-pulsé (les pulses ont une durée et une puissance fixe, mais la puissance est considérable et la durée extrêmement brève) ; et la puissance : de quelques mW à des dizaines de milliers de watts.
- l'IPL : Intense pulse light.
La différence fondamentale entre laser et IPL réside dans le fait que IPL peut délivrer des centaines voire des milliers de couleurs à la fois alors que le laser ne délivre qu'une seule longueur d'onde. Ces machines permettent de choisir la longueur d'onde adaptée au problème à traiter, rien qu'en changeant le filtre. On les appelle aussi « non coherent light source ».
Il s'agit d'émission de pulses de lumière de haute intensité globale.
Cette technologie peut délivrer un large spectre de longueurs d'onde qui sont absorbées par de multiples chromophores. On peut traiter de grandes surfaces à la fois.
- les LED : Light emitting diode (Photomodulation (LED, Light Emitting Diode voir "The newest Médical breakthrough for skin renewal and Shrinking pores (2004)")
La LED : la LED émet généralement de la lumière à basse intensité de quelques milliwatt et à une longueur d'onde de 590+/- 10 à 20 nm, on les classe dans la catégorie des lasers à faible puissance (1 à quelques dizaines de mW).

La présente invention peut également être mise en oeuvre à l'aide de substances émettant de la lumière verte.

Ces substances sont plus particulièrement des métabolites ou actifs émettant de la lumière entre 500 et 580 nm sous exposition aux UV, par exemple, la vitamine B2 qui se dégrade en lumiflavine qui fluoresce dans le vert, la baicailine en milieu acide, la fisetine, le dipyridamole, les pigments phosphorescents verts, les minéraux. Elles sont choisies notamment parmi la lumiflavine, la baicailine en milieu acide, la fisetine, le dipyridamole, les pigments phosphorescents verts et les minéraux

Ces sources de lumière verte sont utilisées en quantité ou intensité suffisante pour assurer un flux d'émission de lumière de 1 à quelques dizaines de mV.

L'homme du métier adaptera la durée d'exposition à la lumière verte selon les caractéristiques de la source lumineuse et l'effet recherché, à titre purement indicatif, les zones cutanées à traiter pourront recevoir une puissance lumineuse d'au moins 500 à 1500 mJ/cm², et préférentiellement, comprise entre 500 et 90000 mJ/cm².

La source de lumière verte pourra encore être une substance phosphorescente ou fluorescente verte.
Par "pigments phosphorescents verts", on entend les pigments phosphorescents classiques listés ci-après mais aussi toute substance cosmétiquement acceptable qui émet une lumière verte phosphorescente d'une longueur d'onde comprise entre environ 500 et 580 nm.
La présence de pigments phosphorescents fournit une source continue de lumière verte à la composition, la phosphorescence est activée par l'exposition aux UV classiquement présent dans la lumière du jour et son effet dure plusieurs heures.
Différents composés sont connus pour avoir la propriété de phosphorer dans le vert comprenant sans limitation :
- le sulfide de zinc (dopé avec du cuivre et/ou du manganèse),
- l'aluminate de strontium
- calcite phosphorescent
Des oxydes céramiques dopés avec des éléments de terre rare tels que l'yttrium dopé Al, le galium, le terbium dopé Sr ou le dysprosyum (Y₃(Al,Ga)SO₂, Y₂SiO₅, SrGa₂S₄:Eu)
- des boro-silicates de zinc (verre) dopés avec du cuivre ou des éléments terreux rares
- les oxydes de terre rare.

En complément ou en remplacement des sources de lumières phosphorescentes, des sources de lumière fluorescentes dans le vert peuvent être utilisées.
Les sources de lumière verte fluorescente préférées sont les minéraux fluorescents.

A titre d'exemple et sans caractère limitatif, on peut citer : l'andalousite et la chiastolite (aluminum silicate); amblygonite (lithium aluminium phosphorate basique); phenakite (beryllium silicate); variscite (aluminium phosphate aqueux); serpentine (basic magnésium silicate); amazonite (potassium aluminium silicate); améthyste (silicone dioxide); chrysoberyl (l'oxyde beryllium aluminum); turquoise (aluminium phosphate basique comprenant du cuivre); tourmaline incolore, jaune ou rose (borosilicate); ambre (succinite/résines); opale (silicone dioxide aqueux); cerussite (lead carbonate); fuchsite (potassium aluminium silicate); diopside (calcium magnésium silicate); ulexite (sodium calcium borate aqueux); aragonite (calcium carbonate); et willemite (zinc silicate).

On peut encore citer les réseaux de polymères qui agissent comme des prismes et ont pour propriété, en choisissant l'indice de réfraction adapté, d'émettre de la lumière verte lorsqu'ils sont éclairés par de la lumière blanche.

On peut encore favoriser l'accès de lumière verte au niveau de la peau en appliquant des compositions colorées vertes qui ont pour rôle de filtrer la lumière pour ne laisser passer que la lumière verte, tels que les oxydes de chrome, la chlorophylle, la kermaline, la tourmaline, des poudres de pierres précieuses et semi-précieuses vertes (émeraude...).

Ces substances peuvent être utilisées à des concentrations suffisantes pour assurer le passage d'une quantité de lumière verte capable d'activer la L-amino acide oxydase, par exemple, comprises entre 0,01 % et 20%.

L'invention a encore pour objet une composition comprenant au moins un substrat de la L-amino acide oxydase et au moins un composé choisi parmi les actifs émettant de la lumière entre 500 et 580 nm sous exposition aux UV, les substances phosphorescentes ou fluorescentes vertes et les substances filtrant la lumière pour ne laisser passer que la lumière verte, ces composés étant choisis parmi la baicailine en milieu acide, la fisetine, le dipyridamole, les pigments et les minéraux.

L'invention concerne également une composition sous forme d'un produit de combinaison pour une utilisation simultanée, séparée ou étalée dans le temps, comprenant au moins un substrat de la L-amino acide oxydase tel que décrit précédemment, et au moins un composé choisi parmi les actifs émettant de la lumière entre 500 et 580 nm sous exposition aux UV, les substances phosphorescentes ou fluorescentes vertes et les substances filtrant la lumière pour ne laisser passer que la lumière verte; de préférence, l'actif émettant de la lumière sous exposition aux UV est choisi parmi la lumiflavine, la baicailine en milieu acide, la fisetine, le dipyridamole, les pigments phosphorescents verts, les minéraux.

Les compositions et procédés selon l'invention permettent de résoudre les problèmes d'inocuité et de formulation potentiels engendrés par l'utilisation d'enzymes, dont l'activité ne doit pas être altérée par la formulation.

L'invention a également pour objet un procédé cosmétique pour améliorer l'apparence de la peau, améliorer l'éclat du teint, atténuer les irrégularités visibles ou tactiles de la surface de la peau, en particulier pour atténuer les rides et ridules et/ou les taches cutanées et/ou lisser la peau et/ou éclaircir le teint, comprenant l'application topique d'une composition comprenant au moins un substrat de la L-amino acide oxydase et l'exposition de la zone sur laquelle ladite composition a été appliquée à une lumière verte de longueur d'onde comprise entre 500 et 580 nm.

L'invention a également pour objet un procédé cosmétique pour lutter contre le vieillissement des cheveux, le ralentissement de leur pousse, leur grisonnement, la diminution de leur diamètre, de leur vigueur, comprenant l'application topique d'une composition comprenant au moins un substrat de la L-amino acide oxydase et l'exposition de la zone sur laquelle ladite composition a été appliquée à une lumière verte de longueur d'onde comprise entre 500 et 580 nm.

Pour la mise en oeuvre des procédés selon l'invention, la source de lumière verte est choisie parmi les lasers, l'IPL, les LED, les métabolites ou actifs émettant de la lumière entre 500 et 580 nm sous exposition aux UV, les substances phosphorescentes ou fluorescentes vertes. Le procédé selon l'invention pourra comprendre en outre une étape d'exposition aux UV. Selon un autre de ses objets, la présente invention se rapporte à un kit comprenant :
a. une composition comprenant au moins un substrat d'une enzyme dont l'activité peut être augmentée par une émission lumineuse verte, la L-amino acide oxydase ;
b. une source de lumière verte, la source de lumière verte étant une substance émettant de la lumière verte formulée dans une autre composition topique que celle contenant ledit substrat de la L-amino acide oxydase.

La mise en oeuvre du procédé peut consister à réaliser les étapes a et b de façon simultanée, séparée ou bien décalée dans le temps.

En particulier, ledit substrat de la L-amino acide oxydase est choisi parmi leucine, la sérine, la thréonine, la glycine, l'alanine, la valine, la phénylalanine, la tyrosine, la lysine, l'arginine, la cystéine, l'aspartate, le glutamate, la citruline, l'ornithine, l'acide diaminopimelique, la penicilamine, l'acide α-amino butyrique.

Ladite composition répond aux caractéristiques techniques décrites ci-avant.

La source de lumière verte est choisie parmi les métabolites ou actifs émettant de la lumière entre 500 et 580 nm sous exposition aux UV, les substances phosphorescentes ou fluorescentes vertes
Ainsi, la source de lumière verte est une substance émettant de la lumière verte formulée dans une autre composition topique destinée à être appliquée de façon décalée (avant ou après).

Un autre kit peut encore être mis en oeuvre à l'aide d'un patch, notamment iontophorétique, imbibé d'au moins un substrat de la L-amino acide oxydase et muni de diodes émettant une lumière verte.

La mise en oeuvre du procédé selon l'invention pourra en outre comprendre une étape préalable ou concomitante aux étapes a et/ou b de traitement de la peau visant à améliorer la pénétration du substrat, par exemple en refroidissant la peau, par iontophorèse, par un système occlusif.

On peut également favoriser la pénétration du substrat en pratiquant un peeling chimique ou mécanique préalable sur la zone à traiter.

### Exemple 1- activation de la L-amino acide oxydase par de la lumière verte

L'exemple suivant mesure l'effet stimulateur de la lumière verte (500-560 nm) sur l'activité du chromophore enzymatique, la L-amino oxydase. L'exposition de cette enzyme à la lumière verte stimule la transformation des acides aminés en cétoacides.

### Description du protocole

Principe générale : Le principe de ces travaux est de comparer l'activité de l'amino acide oxydase avec ou sans exposition préalable sous une source de lumière entre 500-560 nm.

### Matériels et réactifs

- source, avec fibre optique (diamètre interne 3mm)
- Photomètre IL-1700 + sonde SED 033 (#6600).
- la L-amino acide oxydase : Sigma, réf. A-9253 (lot 064K0773) à 0,39 units/mg solid.
- la D-aminoacide oxydase : Sigma, réf. A-5222-100UN (lot 115K1101) à 2,3 units/mg solid.

### Mode exposition des enzymes

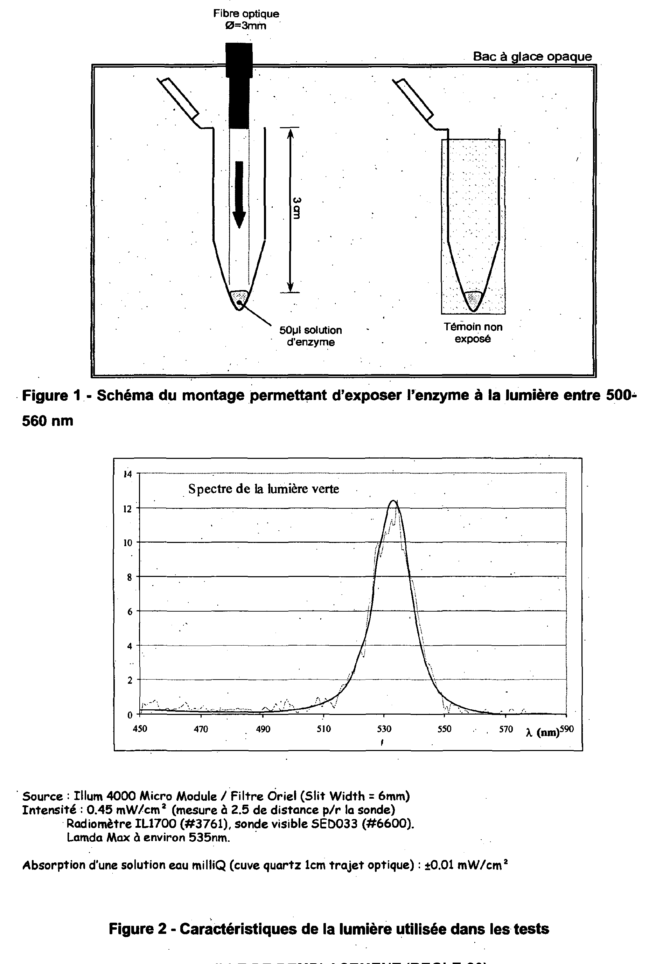

L'enzyme (forme poudre déshydratée ou solution d'enzyme) est disposée dans un tube Ependorf conique. La quantité est ajustée de manière à ce que l'ensemble des enzymes soit soumis aux expositions lumineuses (longueur d'ondes comprises entre 490 et 585 nm) par une fibre optique de diamètre 3 mm. L'exposition lumineuse est continue et l'énergie fournie par la lumière est 0.45mW/cm² (voir figure 1 : schéma de montage et figure 2: caractéristiques de la lumière utilisée dans les tests).
Les tubes Ependorf sont mis dans un bac à glace pendant toute la durée de l'exposition afin d'éviter un effet de la température. L'enzyme témoin (non exposée à la lumière entre 500-560 nm) est préparée au même moment, de la même manière et protégée de la lumière par support empêchant l'apport de lumière extérieure (papier aluminium).

### Mesure d'activité de l'enzyme

L'activité de l'enzyme est mesurée par chimiluminescence.
Le substrat de l'enzyme (L-Leucine et ou D-Alanine) est incubé différents temps avec l'amino acide oxydase dans un milieu tamponné spécifique. La formation du produit d'hydrolyse H₂O₂ est suivie par chimiluminescence en présence de peroxydase et isoluminol selon le protocole de dosage de l'activité de cet enzyme (E.C.1.4.3.2) décrit dans « The Worthington manual », Enzymes and related biochemicals - Editor : Charles C. Worthington 1988, pages 35-37). Le produit d'hydrolyse H₂O₂ qui en est issu est dosé par chimiluminescence avec le mélange réactionnel présence de peroxydase et isoluminol réactionnel préparé comme suit :

### Mélange réactionnel de chimiluminescence :

Peser 34,8 mg d'Isoluminol et les solubiliser sous agitation magnétique dans environ 50 ml de tampon Borate 100 mM. Peser 2 mg de Microperoxydase et les solubiliser sous agitation magnétique dans environ 5 ml de tampon Borate 100 mM. Les deux réactifs sont alors mélangés dans une fiole jaugée de 200 ml dont le volume est ajusté par du tampon Borate (rincer 3 fois chaque bécher avec du tampon). Les 200 ml sont placés dans un flacon de 500 ml. 200 ml de tampon borate y sont alors à nouveau ajoutés. Le mélange réactionnel, dont le volume final est alors de 400 ml est homogénéisé par agitation magnétique puis placé à l'obscurité à température ambiante au moins 24 heures avant emploi.

La lecture de luminescence se fait à l'aide du luminomètre « Fluoroskan Ascent FL » de Thermo Labsystems.

### Résultats : Activation enzymatique spécifique de la L-amino acide oxydase

- Influence de la lumière verte sur la D-amino acide oxydase.

**D amino oxydase**

| Condition | **Témoin** | **lumiere verte** |
|---|---|---|
| luminescence | 577,5 | 568,2166667 |

- Influence de la lumière verte sur la L-amino acide oxydase.

**L amino acide oxydase**

| Condition | **Témoin** | **lumiere verte** |
|---|---|---|
| luminescence | 342,26667 | 409,8777778 |

### Conclusion

Une exposition prolongée à la lumière verte ne modifie pas l'activité de la D-amino acide oxydase de façon significative. En revanche, il est observé qu'une exposition de 60 minutes induit une activation spécifique de la L amino oxydase.

### Exemple 2- cinétique d'activation de la L amino acide oxydase

Plusieurs mesures sont réalisées après différentes durées d'exposition selon le protocole décrit dans l'exemple 1 et récapitulées dans le tableau suivant :

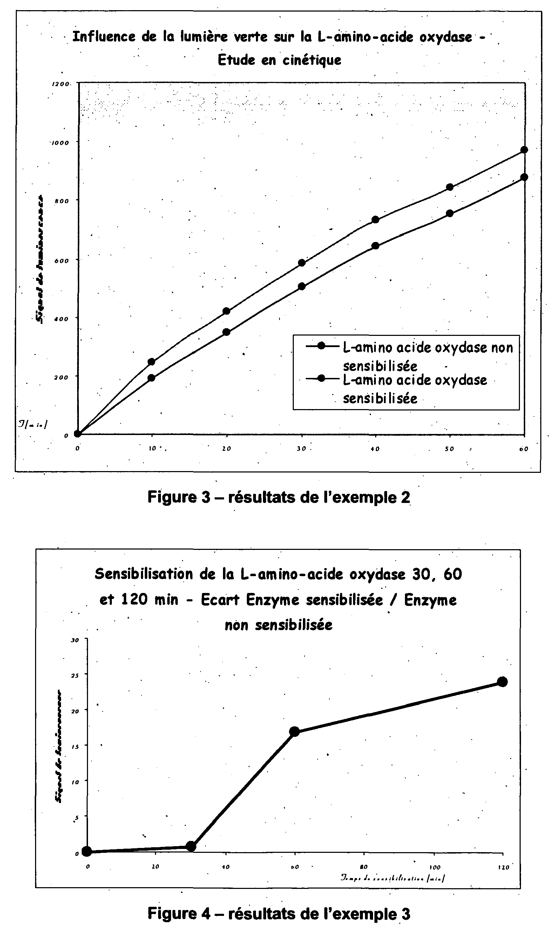

Les résultats figurent dans le tableau ci-dessous et sont représentés en figure 3 (la L-amino acide oxydase sensiblisée est celle exposée à la lumière verte).

| **Temps d'analyse** | **Témoin** | **lumière verte** |
|---|---|---|
| ***0*** | 0 | 0,0 |
| ***10*** | 191,56667 | 248,0 |
| ***20*** | 349,66667 | 420,8 |
| ***30*** | 502,6 | 586,5 |
| ***40*** | 646,46667 | 735,2 |
| ***50*** | 756,26667 | 844,0 |
| ***60*** | 876,36667 | 970,9 |

### Conclusion

La cinétique d'activation de la L amino acide oxydase est augmentée par une exposition à la lumière entre 500-560 nm.

### Exemple 3- activation de la L-amino acide oxydase selon le temps d'exposition : effet dose réponse.

L'essai est mis en oeuvre dans les mêmes conditions expérimentales que l'exemple 1 en suivant le protocole du forunisseur.

| **Temps de stimulation** | **Témoin** | **lumière verte** | **Ecart** |
|---|---|---|---|
| ***0*** | 0 | 0,0 | 0 |
| ***30*** | 409,9 | 412,8 | 0,7 |
| ***60*** | 349,7 | 420,8 | 16,9 |
| ***120*** | 275,4 | 361,7 | 23,8 |

Les résultats figurent dans le tableau ci-dessous et sont représentés en figure 4.

### Conclusion

On constate que plus l'exposition de l'enzyme est longue plus son activité est importante. Le diagramme suivant montre le rapport entre l'activité de l'enzyme exposée et celle de l'enzyme témoin.

### Exemple 4 - Compositions

| *Crème de régénération épidermique (émulsion huile dans eau)* | |
|---|---|
| Ammonium Polyacryldimethyltauramide (Hostacerin AMPS de Clariant) | 1.00% |
| Cyclohexasiloxane | 5.0% |
| Glycerin | 1.70% |
| Stearyl alcohol | 0.30% |
| Glyceryl stearate / PEG-100 stearate | 0.70% |
| Dimyristyl tartrate / cetearyl alcohol / C12-15 pareth-7 / PPG-25 laureth-25 | 0.50% |
| Gomme de Xanthan | 0,20% |
| L-Alanine | 0.5% |
| Conservateurs | 0.50% |
| Eau | qsp 100 |

| *Crème de régénération épidermique (émulsion huile dans eau)* | |
|---|---|
| Ammonium Polyacryldimethyltauramide (Hostacerin AMPS de Clariant) | 1.00% |
| Cyclohexasiloxane | 5.0% |
| Glycerin | 1.70% |
| Stearyl alcohol | 0.30% |
| Glyceryl stearate / PEG-100 stearate | 0.70% |
| Dimyristyl tartrate / cetearyl alcohol / C12-15 pareth-7 / PPG-25 laureth-25 | 0.50% |
| Gomme de Xanthan | 0,20% |
| L-Leucine | 0.5% |
| Conservateurs | 0.50% |
| Eau | qsp 100 |

Ces crèmes sont appliquées sur le visage et/ou sur le corps juste avant une exposition prolongé (30 mn à 1h30) sous un rayonnement de longueur d'onde comprise entre 500 et 560 nm.

### Patch iontophorétique

Un patch de référence commercial lontopatchTM (Travanti Pharma, Mendota Heights, MN, USA) est appliqué sur une zone préalablement traitée avec l'une des crèmes ci-dessus.
Il est ensuite relié à un courant électrique pour délivrer un courant galvanique généré par une différence de potentiel de 1V et comportant deux électrodes, une anode en Zn et une cathode en AgCl et est exposé sous une source de lumière verte.
Ce traitement est réalisé à raison d'une fois par jour pendant 30 à 45 minutes.

### Masque vert

| Phase huileuse : | |
|---|---|
| Octyl dodecanol | 6% |
| Huile de noyau d'abricot | 6% |
| Triglycerides | 5% |
| Kaolin | 3% |
| Alcool cétylique | 2% |
| Acetate de vitamine E | 0.5% |
| Huile de palm hydrogénée | 6% |
| Fraction liquide de beurre de Karité | 5% |

| Phase aqueuse : | |
|---|---|
| Gomme de Xanthane | 0.4% |
| Cocoate de sucrose/stearate de de sorbitan(melande vendu par la société ICL sous la | |
| denomination Arlaton 21121 | 5.5% |
| Glycerine | 3% |
| Dipyridamole | 0.30% |
| Cuivre Chlorophyllin | 0.20% |
| Ethanol | 5% |
| L_Alanine | 0.5% |
| Parfum | 0.3% |
| Conservateur | qs |
| Eau | qsp100 |

Ce masque est appliqué sur les zones de la peau à traiter, puis le sujet est placé sous une source de lumière blanche (naturelle ou électrique) pendant 45 minutes à 1 heure.

## Revendications

1. Utilisation non-thérapeutique d'une lumière verte de longueur d'onde comprise entre 500 et 580 nm pour activer la L-amino acide oxydase en présence d'au moins un de ses substrats.

2. Utilisation non-thérapeutique selon la revendication 1, **caractérisée en ce que** ledit substrat est un amino acide ou une molécule comprenant une fonction amine, tel que qu'il comporte un carbone en alpha du groupe acide et un groupe d'amine primaire.

3. Utilisation non-thérapeutique selon la revendication 2, **caractérisée en ce que** ledit substrat est choisi parmi la leucine, la sérine, la thréonine, la glycine, l'alanine, la valine, la phénylalanine, la tyrosine, la lysine, l'arginine, la cystéine, l'aspartate, le glutamate, la citruline, l'ornithine, l'acide diaminopimelique, la penicilamine, l'acide α-amino butyrique.

4. Utilisation non-thérapeutique selon la revendication 3, **caractérisée en ce que** ledit substrat est présent à une concentration comprise entre 0,001 et 10%.

5. Utilisation non-thérapeutique selon l'une quelconque des revendications précédentes, **caractérisée en ce que** ledit substrat est formulé dans une composition topique.

6. Utilisation non-thérapeutique selon l'une quelconque des revendications précédentes, **caractérisée en ce que** la source de lumière verte est choisie parmi les lasers, l'IPL, les LED, les métabolites ou actifs émettant de la lumière entre 500 et 580 nm sous exposition aux UV, les substances phosphorescentes ou fluorescentes vertes.

7. Utilisation non-thérapeutique selon la revendication 6, **caractérisée en ce que** lesdits métabolites ou actifs émettant de la lumière entre 500 et 580 nm sous exposition aux UV sont choisis parmi la vitamine B2, la baicailine en milieu acide, la fisetine, le dipyridamole, les pigments, les minéraux.

8. Utilisation non-thérapeutique selon la revendication 6, **caractérisée en ce que** ladite source de lumière verte produit une puissance lumineuse comprise entre 500 et 90000 mJ/cm².

9. Utilisation non-thérapeutique selon l'une quelconque des revendications précédentes, pour activer le métabolisme des cellules de la peau, en particulier, des fibroblastes, des kératinocytes et des mélanocytes et/ou activer et/ou réguler la physiologie de ces cellules.

10. Utilisation non-thérapeutique selon la revendication 9, pour diminuer la formation et/ou en stimuler l'élimination de composants cellulaires endommagés.

11. Utilisation non-thérapeutique selon la revendication 9, pour stimuler le renouvellement des cellules de l'épiderme et la régénération cutanée.

12. Utilisation non-thérapeutique selon l'une quelconque des revendications précédentes, pour donner à la peau et aux cheveux un aspect sain, en particulier, unifier le teint, raviver son éclat, éviter son aspect jaune ou terne ; prévenir et/ou traiter les taches cutanées ; atténuer les irrégularités visibles et/ou tactiles de la surface de la peau, lui donner un relief plus uniforme et la lisser, prévenir et/ou traiter les rides et ridules ; redonner plus d'épaisseur et d'élasticité à la peau.

13. Utilisation non-thérapeutique selon l'une quelconque des revendications 1 à 11, pour lutter contre les altérations de la peau liées au vieillissement, prévenir et/ou traiter les signes cutanés du vieillissement de la peau et les signes du vieillissement des cheveux.

14. Utilisation non-thérapeutique selon la revendication 13, pour prévenir et/ou traiter le relâchement de la peau, l'apparition de rides, de taches cutanées, un jaunissement du teint.

15. Utilisation non-thérapeutique selon la revendication 13, pour prévenir et/ou traiter la chute des cheveux, le ralentissement de leur pousse, leur grisonnement, la diminution de leur diamètre, de leur vigueur.

16. Composition comprenant au moins un substrat de la L-amino acide oxydase et au moins un composé choisi parmi les actifs émettant de la lumière entre 500 et 580 nm sous exposition aux UV, les substances phosphorescentes ou fluorescentes vertes et les substances filtrant la lumière pour ne laisser passer que la lumière verte, **caractérisée en ce que** les actifs émettant de la lumière entre 500 et 580 nm sous exposition aux UV, les substances phosphorescentes ou fluorescentes vertes sont choisis parmi la baicailine en milieu acide, la fisetine, le dipyridamole, les pigments, les minéraux

17. Composition selon la revendication 17, **caractérisée en ce que** ledit substrat est choisi parmi la leucine, la sérine, la thréonine, la glycine, l'alanine, la valine, la phénylalanine, la tyrosine, la lysine, l'arginine, la cystéine, l'aspartate, le glutamate, la citruline, l'ornithine, l'acide diaminopimelique, la penicilamine, l'acide α-amino butyrique.

18. Procédé cosmétique pour améliorer l'apparence de la peau, améliorer l'éclat du teint, atténuer les irrégularités visibles ou tactiles de la surface de la peau, en particulier pour atténuer les rides et ridules et/ou les taches cutanées et/ou lisser la peau et/ou éclaircir le teint, comprenant l'application topique d'une composition comprenant au moins un substrat de la L-amino acide oxydase et l'exposition de la zone sur laquelle ladite composition a été appliquée à une lumière verte de longueur d'onde comprise entre 500 et 580 nm.

19. Procédé cosmétique pour lutter contre le vieillissement des cheveux, le ralentissement de leur pousse, leur grisonnement, la diminution de leur diamètre, de leur vigueur, comprenant l'application topique d'une composition comprenant au moins un substrat de la L-amino acide oxydase et l'exposition de la zone sur laquelle ladite composition a été appliquée à une lumière verte de longueur d'onde comprise entre 500 et 580 nm.

20. Procédé selon la revendication 19 ou 20, **caractérisé en ce que** la source de ladite lumière verte est choisie parmi les lasers, l'IPL, les LED, les métabolites ou actifs émettant de la lumière entre 500 et 580 nm sous exposition aux UV, les substances phosphorescentes ou fluorescentes vertes.

21. Kit comprenant :
a. une composition comprenant au moins un substrat de la L-amino acide oxydase ;
b. une source de lumière verte, la source de lumière verte étant une substance émettant de la lumière verte formulée dans une autre composition topique que celle contenant ledit substrat de la L-amino acide oxydase.

22. Kit selon la revendication 22, **caractérisé en ce que** ledit substrat est choisi parmi la leucine, la sérine, la thréonine, la glycine, l'alanine, la valine, la phénylalanine, la tyrosine, la lysine, l'arginine, la cystéine, l'aspartate, le glutamate, la citruline, l'ornithine, l'acide diaminopimelique, la penicilamine, l'acide α-amino butyrique.

23. Kit selon la revendication 22 ou 23, **caractérisé en ce que** ladite source de lumière verte est choisie parmi les métabolites ou actifs émettant de la lumière entre 500 et 580 nm sous exposition aux UV, les substances phosphorescentes ou fluorescentes vertes.

## Patentansprüche

1. Nicht-therapeutische Verwendung eines grünen Lichts einer Wellenlänge zwischen 500 und 580 nm zum Aktivieren der L-Aminosäure-Oxidase in Gegenwart mindestens eines ihrer Substrate.

2. Nicht-therapeutische Verwendung nach Anspruch 1, **dadurch gekennzeichnet, dass** es sich bei dem Substrat um eine Aminosäure oder ein eine Aminfunktion umfassendes Molekül handelt, derart, dass es einen Kohlenstoff in alpha-Stellung zu der Säuregruppe und eine primäre Amingruppe aufweist.

3. Nicht-therapeutische Verwendung nach Anspruch 2, **dadurch gekennzeichnet, dass** das Substrat aus Leucin, Serin, Threonin, Glycin, Alanin, Valin, Phenylalanin, Tyrosin, Lysin, Arginin, Cystein, Aspartat, Glutamat, Citrullin, Ornithin, Diaminopimelinsäure, Penicillamin, α-Aminobuttersäure ausgewählt ist.

4. Nicht-therapeutische Verwendung nach Anspruch 3, **dadurch gekennzeichnet, dass** das Substrat in einer Konzentration zwischen 0,001 und 10% vorhanden ist.

5. Nicht-therapeutische Verwendung nach einem der vorhergehenden Ansprüche, **dadurch gekennzeichnet, dass** das Substrat in einer topischen Zusammensetzung formuliert ist.

6. Nicht-therapeutische Verwendung nach einem der vorhergehenden Ansprüche, **dadurch gekennzeichnet, dass** die Quelle von grünem Licht aus Lasern, IPL, LED, Metaboliten oder Wirkstoffen, die Licht zwischen 500 und 580 nm unter Belichtung mit UV emittieren, grün phosphoreszierenden oder fluoreszierenden Substanzen ausgewählt ist.

7. Nicht-therapeutische Verwendung nach Anspruch 6, **dadurch gekennzeichnet, dass** die Metaboliten oder Wirkstoffe, die Licht zwischen 500 und 580 nm unter Belichtung mit UV-Licht emittieren, aus Vitamin B2, Baicailin in saurem Medium, Fisetin, Dipyridamol, Pigmenten, Mineralien ausgewählt sind.

8. Nicht-therapeutische Verwendung nach Anspruch 6, **dadurch gekennzeichnet, dass** die Quelle von grünem Licht eine Lichtstärke zwischen 500 und 90000 mJ/cm² erzeugt.

9. Nicht-therapeutische Verwendung nach einem der vorhergehenden Ansprüche zum Aktivieren des Metabolismus der Hautzellen, insbesondere der Fibroblasten, der Keratinozyten und der Melanozyten, und/oder zum Aktivieren und/oder Regulieren der Physiologie dieser Zellen.

10. Nicht-therapeutische Verwendung nach Anspruch 9 zur Verringerung der Bildung und/oder zum Stimulieren der Beseitigung von beschädigten zellulären Komponenten darin.

11. Nicht-therapeutische Verwendung nach Anspruch 9 zum Stimulieren der Erneuerung der Zellen der Epidermis und der Regeneration der Haut.

12. Nicht-therapeutische Verwendung nach einem der vorhergehenden Ansprüche, um der Haut und den Haaren ein gesundes Aussehen zu verleihen, insbesondere die Hautfarbe zu vereinheitlichen, ihren Glanz wiederzubeleben, ihr gelbes oder glanzloses Aussehen zu vermeiden; Hautmakel vorzubeugen und/oder zu behandeln; sichtbare und/oder fühlbare Unregelmäßigkeiten der Oberfläche der Haut abzuschwächen, ihr ein gleichmäßigeres Profil zu verleihen und sie zu glätten, Falten und Linien vorzubeugen und/oder zu behandeln; der Haut mehr Dicke und mehr Elastizität wiederzugeben.

13. Nicht-therapeutische Verwendung nach einem der Ansprüche 1 bis 11, um Veränderungen der Haut in Zusammenhang mit dem Altern zu bekämpfen, kutane Anzeichen für die Hautalterung und Anzeichen für das Altern der Haare vorzubeugen und/oder zu behandeln.

14. Nicht-therapeutische Verwendung nach Anspruch 13, zum Vorbeugen und/oder Behandeln von Erschlaffung der Haut, des Auftretens von Falten, Hautmakeln, Gelbfärbung der Hautfarbe.

15. Nicht-therapeutische Verwendung nach Anspruch 13 zum Vorbeugen und/oder Behandeln von Haarausfall, von Verlangsamung ihres Wachstums, ihrer Vergrauung, der Verringerung ihres Durchmessers, ihrer Kraft.

16. Zusammensetzung, umfassend mindestens ein Substrat der L-Aminosäure-Oxidase und mindestens eine Verbindung, ausgewählt aus Wirkstoffen, die Licht zwischen 500 und 580 nm unter Belichtung mit UV emittieren, grün phosphoreszierenden oder fluoreszierenden Substanzen und Substanzen, die das Licht filtern, so dass sie nur grünes Licht durchlassen, **dadurch gekennzeichnet, dass** die Wirkstoffe, die Licht zwischen 500 und 580 nm unter Belichtung mit UV emittieren, die grün phosphoreszierenden oder fluoreszierenden Substanzen aus Baicailin in saurem Medium, Fisetin, Dipyridamol, Pigmenten, Mineralien ausgewählt sind.

17. Zusammensetzung nach Anspruch 17, **dadurch gekennzeichnet, dass** das Substrat aus Leucin, Serin, Threonin, Glycin, Alanin, Valin, Phenylalanin, Tyrosin, Lysin, Arginin, Cystein, Aspartat, Glutamat, Citrullin, Ornithin, Diaminopimelinsäure, Penicillamin, α-Aminobuttersäure ausgewählt ist.

18. Kosmetisches Verfahren zum Verbessern des Aussehens der Haut, Verbessern des Glanzes der Hautfarbe, Abschwächen sichtbarer oder fühlbarer Unregelmäßigkeiten der Oberfläche der Haut, insbesondere um Linien und Falten und/oder Hautmakel abzuschwächen und/oder die Haut glätten und/oder die Hautfarbe aufzuhellen, umfassend die topische Anwendung einer Zusammensetzung, die mindestens ein Substrat der L-Aminosäure-Oxidase umfasst, und das Belichten des Bereichs, auf den die Zusammensetzung aufgetragen wurde, mit grünem Licht einer Wellenlänge zwischen 500 und 580 nm.

19. Kosmetisches Verfahren zum Bekämpfen des Alterns der Haare, der Verlangsamung ihres Wachstums, ihrer Vergrauung, der Verringerung ihres Durchmessers, ihrer Kraft, umfassend die topische Anwendung einer Zusammensetzung, die mindestens ein Substrat der L-Aminosäure-Oxidase umfasst, und das Belichten des Bereichs, auf den die Zusammensetzung aufgetragen wurde, mit grünem Licht einer Wellenlänge zwischen 500 und 580 nm.

20. Verfahren nach Anspruch 19 oder 20, **dadurch gekennzeichnet, dass** die Quelle des grünen Lichts aus Lasern, IPL, LED, Metaboliten oder Wirkstoffen, die Licht zwischen 500 und 580 nm unter Belichtung mit UV emittieren, grün phosphoreszierenden oder fluoreszierenden Substanzen ausgewählt ist.

21. Kit, umfassend:
a. eine Zusammensetzung, die mindestens ein Substrat der L-Aminosäure-Oxidase umfasst;
b. eine Quelle von grünem Licht,
wobei es sich bei der Quelle von grünem Licht um eine grünes Licht emittierende Substanz handelt, die in einer anderen topischen Zusammensetzung als diejenige, die das Substrat der L-Aminosäure-Oxidase enthält, formuliert ist.

22. Kit nach Anspruch 22, **dadurch gekennzeichnet, dass** das Substrat aus Leucin, Serin, Threonin, Glycin, Alanin, Valin, Phenylalanin, Tyrosin, Lysin, Arginin, Cystein, Aspartat, Glutamat, Citrullin, Ornithin, Diaminopimelinsäure, Penicillamin, α-Aminobuttersäure ausgewählt ist.

23. Kit nach Anspruch 22 oder 23, **dadurch gekennzeichnet, dass** die Quelle von grünem Licht aus Metaboliten oder Wirkstoffen, die Licht zwischen 500 und 580 nm unter Belichtung mit UV emittieren, grün phosphoreszierenden oder fluoreszierenden Substanzen ausgewählt ist.

## Claims

1. Non-therapeutic use of a green light with a wavelength of between 500 and 580 nm for activating L-amino acid oxidase in the presence of at least one of its substrates.

2. Non-therapeutic use according to Claim 1, **characterized in that** the said substrate is an amino acid or a molecule comprising an amine functional group, such that the substrate comprises a carbon in the α position with regard to the acid group and a primary amine group.

3. Non-therapeutic use according to Claim 2, **characterized in that** the said substrate is chosen from leucine, serine, threonine, glycine, alanine, valine, phenylalanine, tyrosine, lysine, arginine, cysteine, aspartate, glutamate, citrulline, ornithine, diaminopimelic acid, penicillamine or α-aminobutyric acid.

4. Non-therapeutic use according to Claim 3, **characterized in that** the said subtrate is present at a concentration of between 0.001 and 10%.

5. Non-therapeutic use according to any one of the preceding claims, **characterized in that** the said substrate is formulated in a topical composition.

6. Non-therapeutic use according to any one of the preceding claims, **characterized in that** the source of green light is chosen from lasers, IPL, LEDs, metabolites or active principles which emit light between 500 and 580 nm on exposure to UV radiation, green phosphorescent substances or green fluorescent substances.

7. Non-therapeutic use according to Claim 6, **characterized in that** the said metabolites or active principles which emit light between 500 and 580 nm on exposure to UV radiation are chosen from vitamin B2, baicailine in acid medium, fisetin, dipyridamole, pigments or minerals.

8. Non-therapeutic use according to Claim 6, **characterized in that** the said source of green light produces a light power of between 500 and 90 000 mJ/cm².

9. Non-therapeutic use according to any one of the preceding claims for activating the metabolism of the cells of the skin, in particular fibroblasts, keratinocytes and melanocytes, and/or activating and/or regulating the physiology of these cells.

10. Non-therapeutic use according to Claim 9 for reducing the formation and/or stimulating the removal of damaged cell components.

11. Non-therapeutic use according to Claim 9 for stimulating the replacement of the cells of the epidermis and cutaneous regeneration.

12. Non-therapeutic use according to any one of the preceding claims for giving a healthy appearance to the skin and hair, in particular unifying the complexion, rekindling its radiance, preventing its yellow or lifeless appearance; preventing and/or treating cutaneous blemishes; toning down visible and/or tactile irregularities of the surface of the skin, giving it a more uniform relief and smoothing it, preventing and/or treating wrinkles and fine lines; restoring greater thickness and elasticity to the skin.

13. Non-therapeutic use according to any one of Claims 1 to 11 for combating detrimental changes to the skin related to ageing, preventing and/or treating cutaneous signs of ageing of the skin and signs of ageing of the hair.

14. Non-therapeutic use according to Claim 13 for preventing and/or treating relaxing of the skin, the appearance of wrinkles or cutaneous blemishes, or yellowing of the complexion.

15. Non-therapeutic use according to Claim 13 for preventing and/or treating loss of hair, the slowing of its growth, its greying, the decrease in the diameter of the individual hairs or the decrease in the vigour of the hair.

16. Composition comprising at least one substrate of L-amino acid oxidase and at least one compound chosen from active principles which emit light between 500 and 580 nm on exposure to UV radiation, green phosphorescent substances, green fluorescent substances and substances which screen light in order to allow only green light to pass through, **characterized in that** the active principles which emit light between 500 and 580 nm on exposure to UV radiation, green phosphorescent substances or green fluorescent substances, are chosen from baicailine in acid medium, fisetin, dipyridamole, pigments or minerals.

17. Composition according to Claim 17, **characterized in that** the said substrate is chosen from leucine, serine, threonine, glycine, alanine, valine, phenylalanine, tyrosine, lysine, arginine, cysteine, aspartate, glutamate, citrulline, ornithine, diaminopimelic acid, penicillamine, or α-aminobutyric acid.

18. Cosmetic method for improving the appearance of the skin, improving the radiance of the complexion, toning down visible or tactile irregularities of the surface of the skin, in particular for toning down wrinkles and fine lines and/or cutaneous blemishes, and/or smoothing the skin and/or lightening the complexion, comprising the topical application of a composition comprising at least one substrate of L-amino acid oxidase and the exposure of the region on which the said composition has been applied to green light with a wavelength of between 500 and 580 nm.

19. Cosmetic method for combating ageing of the hair, the slowing of its growth, its greying, the reduction in the diameter of the individual hairs or the reduction in the vigour of the hair, comprising the topical application of a composition comprising at least one substrate of L-amino acid oxidase and the exposure of the region on which the said composition has been applied to a green light with a wavelength of between 500 and 580 nm.

20. Process according to Claim 19 or 20, **characterized in that** the source of the said green light is chosen from lasers, IPL, LEDs, metabolites or active principles which emit light between 500 and 580 nm on exposure to UV radiation, green phosphorescent substances or green fluorescent substances.

21. Kit comprising:
a. a composition comprising at least one substrate of L-amino acid oxidase;
b. a source of green light,
the source of green light being a substance which emits green light formulated in a topical composition other than that comprising the said substance of L-amino acid oxidase.

22. Kit according to Claim 22, **characterized in that** the said substrate is chosen from leucine, serine, threonine, glycine, alanine, valine, phenylalanine, tyrosine, lysine, arginine, cysteine, aspartate, glutamate, citrulline, ornithine, diaminopimelic acid, penicillamine, or α-aminobutyric acid.

23. Kit according to Claim 22 or 23, **characterized in that** the said source of green light is chosen from metabolites or active principles which emit light between 500 and 580 nm on exposure to UV radiation, green phosphorescent substances or green fluorescent substances.
